# EUROPÄISCHE PATENTANMELDUNG

(11) **EP 3 808 724 A1**
(43) Veröffentlichungstag der Anmeldung: **21.04.2021**
(21) Anmeldenummer: 19020577.3
(22) Anmeldetag: 16.10.2019
(51) Int. Cl.: C07C 29/16, C07C 31/04

(54) **VERFAHREN ZUM HERSTELLEN VON METHANOL MITTELS MEHRSTUFIGER SYNTHESE**

(71) Anmelder: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Oelmann, Tobias, 61118 Bad Vilbel (DE); Gronemann, Veronika, 61184 Karben (DE); Hofmann, Heiko, 63225 Langen (DE); Schuhmann, Timm, 64625 Bensheim (DE)
(74) Vertreter: Dropsch, Holger

(57) **Zusammenfassung**

Es wird ein Verfahren zum Herstellen von Methanol aus Synthesegas mittels mehrstufiger, beispielsweise zweistufiger, heterogen-katalytischer Methanolsynthese vorgeschlagen, wobei das in jeder Synthesestufe gebildete Methanol-Produkt durch Kondensation abgetrennt wird und das verbliebene Restgas der stromabwärts angeordneten Synthesestufe aufgegeben oder nach Abtrennen eines Spülstroms als Recyclestrom zu der ersten Synthesestufe zurückgeführt wird. Erfindungsgemäß wird aus dem Synthesegas-Frischgas ein Teilstrom abgetrennt und als Bypassstrom in den zweiten Methanolsynthesereaktor eingeleitet.

## Beschreibung

### Gebiet der Erfindung

Die Erfindung betrifft ein Verfahren zur Herstellung von Methanol durch heterogen-katalysierte Umsetzung von Wasserstoff und Kohlenoxide umfassendem Synthesegas an festen, körnigen Katalysatoren zur Methanolsynthese, die in mehreren, hintereinandergeschalteten Festbettreaktoren angeordnet sind. Die Erfindung betrifft ferner eine Anlage zur Durchführung eines solchen Herstellungsverfahrens.

### Stand der Technik

Verfahren zur industriellen Herstellung von Methanol durch heterogen-katalytische Umsetzung von Synthesegas, also Gemischen von Wasserstoff und Kohlenoxiden, sind der Fachwelt seit langem bekannt. In Ullmann's Encyclopedia of Industrial Chemistry, Sixth Edition, 1998 Electronic Release, Kapitel "Methanol", Unterkapitel 5.2 "Synthesis" werden verschiedene Grundverfahren zur Herstellung von Methanol durch katalytische Umsetzung von Wasserstoff und Kohlenstoffoxide enthaltendem Synthesegas beschrieben, bei denen solche Reaktoren eingesetzt werden.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor (WCR) und dann einem gasgekühlten Reaktor (GCR) zugeführt wird, in welchen das Synthesegas jeweils an einem kupferbasierten Festbett-Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280 °C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.

In der wassergekühlten Reaktorstufe wird üblicherweise der Hauptumsatz des Synthesegases (CO, CO₂, H₂) erzielt und der größte Teil der Reaktionswärme abgeführt, während in der gasgekühlten Stufe bei milderen Bedingungen ein dennoch erheblicher Teil des Synthesegases umgesetzt wird.

In manchen mehrstufigen Anlagenkonfigurationen zur Methanolsynthese wird zusätzlich eine Zwischenkondensationsstufe zwischen den einzelnen Reaktionsstufen vorgesehen, um den Anteil der gebildeten Reaktionsprodukte (vorwiegend Methanol und Wasser) im Einsatzgas zur nachfolgenden Reaktionsstufe zu verringern. Dies bewirkt sowohl eine günstige Verschiebung der Gleichgewichtslage der Methanolbildungsreaktion in Richtung auf das Zielprodukt Methanol als auch eine Verringerung der Raumgeschwindigkeit bzw. Erhöhung der Verweilzeit im nachfolgenden Reaktor, die ebenfalls den Eduktumsatz weiter erhöht. Eine solche Anlagenkonfigurationen wird beispielsweise in der deutschen Patentschrift DE 10 2008 049 622 B4 gelehrt.

Bei dem wassergekühlten Reaktor (WCR) handelt es sich üblicherweise um einen Röhrenreaktor mit entsprechenden Rohrplatten, bei dem der Katalysator in die Rohre gefüllt wird, während die Kühlung mittels siedendem Wasser bzw. Dampferzeugung auf der Mantelseite um die Rohre erfolgt. Im gasgekühlten Reaktor (GCR) erfolgt die Kühlung mit dem Einsatzgas, welches durch die Rohre geführt wird und sich auf dem Weg zur ersten Reaktionsstufe (WCR) erwärmt, während der Katalysator um die Rohre gefüllt wird und die Reaktion auf der Mantelseite des GCR stattfindet. Die Reaktionsstufen sind hinsichtlich ihrer Nennweite mit großen bzw. sehr großen Rohrleitungen verbunden; je nach Anlagenkapazität sind Rohrdurchmesser von bis zu 1 m möglich. Dies ist vor allem auf die großen Gasmengen zurückzuführen, die zu der zweiten Stufe zurückgeführt (Recycle-Gas) und dem Frischgas oder Make-up-Gas, also frischem Synthesegas aus der Gaserzeugung, zugemischt werden. Das sich ergebende Gasgemisch aus Rückführungsgas und Frischgas wird nach erfolgter Vorwärmung im GCR der ersten Reaktionsstufe (WCR) zugeführt. Die Rückführungsgasmenge (Recycle-Gas) ist üblicherweise deutlich größer als die Frischgasmenge und ist vom erzielten Umsatz in der Reaktorsektion abhängig. Das Rückführverhältnis RR (RR = R/F) aus Rückführungsgasmenge (R) zu Frischgasmenge (F) liegt oft über 2 und in einigen Fällen sogar über 3,5. Je niedriger der Umsatz von Synthesegas durch die Reaktorsektion per Durchgang ist, um so höher ist das erforderliche Rückführverhältnis RR, um eine hinreichende Ausbeute zu erzielen. Damit erhöht sich die umlaufende Gasmenge entsprechend, was die Belastung der Reaktoren erhöht und größere Rohrnennweiten der verbindenden Rohrleitungen bedarf und auch zu einem höherem Bedarf an Kompressionsenergie (höhere Durchflussmenge und Druckverlust) führt.

Üblicherweise werden in beiden Synthesereaktoren dieselben Methanolsynthesekatalysatoren auf Kupferbasis verwendet, die als feste, körnige Katalysatoren in Festbettreaktoren eingesetzt werden. Bei dem beschriebenen, zweistufigen WCR-GCR-Verfahren wird der wassergekühlte Reaktor typischerweise mit höherer Synthesegas-Eintrittstemperatur betrieben als ein wassergekühlter Reaktor in einem einstufigen Verfahren zur Methanolsynthese, um Dampf mit höherem Druck bereitstellen zu können. Ferner wird dieser Reaktor mit noch nicht abreagiertem Synthesegas beaufschlagt. Aufgrund der hohen Exothermie der Methanolsynthese ist daher eine sehr gute Temperaturkontrolle des Reaktors notwendig, um eine Überhitzung des Katalysators zu vermeiden, die wesentlich zu seiner vorzeitigen Desaktivierung beiträgt. In der DE-Offenlegungsschrift DE 102010008857 A1 wird daher vorgeschlagen, in den beiden Synthesereaktoren Katalysatoren mit unterschiedlicher Aktivität zu verwenden, wobei in dem Reaktor mit den drastischeren Reaktionsbedingungen ein Katalysator mit niedrigerer Aktivität eingesetzt werden soll, der eine geringere Desaktivierungsgeschwindigkeit und somit höhere Langzeitbeständigkeit aufweist.

Nachteiligig bei diesem Verfahren ist es jedoch, dass Katalysatoren mit unterschiedlicher Aktivität bereitgestellt werden müssen, was die für den Anlagenbetrieb bereitzustellenende Logistik verkompliziert. Ferner besteht weiterhin Bedarf an einer verbesserten Steuerung der einzelnen Synthesereaktoren, insbesondere im Hinblick auf die dort herrschenden Temperaturen. Lokale Übertemperaturen, sog. "hot spots", sind ein wesentlicher Faktor für eine vorzeitige Desaktivierung der eingesetzten Temperaturen.

### Beschreibung der Erfindung

Die Aufgabe der vorliegenden Erfindung besteht daher darin, ein Verfahren und eine Anlage anzugeben, das die beschriebenen Nachteile des Stands der Technik nicht aufweist und das es insbesondere ermöglicht, in einem mehrstufigen Verfahren bzw. einer mehrstufigen Anlage zur Methanolsynthese mit mehreren hintereinandergeschalteten Synthesereaktoren eine gleichmäßigere Belastung der in den einzelnen Reaktoren angeordneten Katalysatoren und dort ferner eine verbesserte Temperaturkontrolle zu erreichen.

Diese Aufgabe wird durch ein Verfahren mit den Merkmalen des Anspruchs 1 gelöst. Weitere Ausgestaltungen der Erfindung ergeben sich aus den Unteransprüchen der jeweiligen Kategorie.

### Erfindungsgemäßes Verfahren:

Verfahren zum Herstellen von Methanol durch Umsetzen eines Wasserstoff und Kohlenoxide enthaltenden Synthesegaseinsatzstroms, umfassend folgende Verfahrensschritte:
(a) Bereitstellen des Wasserstoff und Kohlenoxide enthaltenden Synthesegaseinsatzstroms, Auftrennen des Synthesegaseinsatzstroms in einen Frischgas-Bypassstrom und in einen Frischgas-Feedstrom,
(b) Zusammenführen und Vermischen des Frischgas-Feedstroms mit einem Wasserstoff und Kohlenoxide enthaltenden Recyclestrom zu einem ersten Reaktorfeedstrom,
(c) Einleiten des ersten Reaktorfeedstroms in einen ersten Methanolsynthesereaktor, enthaltend mindestens eine Katalysatorzone mit einem einen für die Methanolsynthese aktiven, festen, körnigen Katalysator, mindestens teilweises Umsetzen des ersten Reaktorfeedstroms in dem ersten Methanolsynthesereaktor unter Methanolsynthesebedingungen,
(d) Ausleiten eines Methanol und Wasser enthaltenden ersten Reaktorproduktstroms aus dem ersten Methanolsynthesereaktor, Abkühlen des ersten Reaktorproduktstroms unter seinen Taupunkt und Zuführen des abgekühlten ersten Reaktorproduktstroms zu einer ersten Phasentrennvorrichtung,
(e) Auftrennen des abgekühlten ersten Reaktorproduktstroms in der ersten Phasentrennvorrichtung in einen ersten Flüssigproduktstrom und einen ersten Restgasstrom, der nicht umgesetzte Synthesegasbestandteile enthält,
(f) Einleiten des ersten Restgasstroms in einen zweiten Methanolsynthesereaktor, enthaltend mindestens eine Katalysatorzone mit einem einen für die Methanolsynthese aktiven, festen, körnigen Katalysator, mindestens teilweises Umsetzen des ersten Restgasstroms in dem zweiten Methanolsynthesereaktor unter Methanolsynthesebedingungen,
(g) Ausleiten eines Methanol und Wasser enthaltenden zweiten Reaktorproduktstroms aus dem zweiten Methanolsynthesereaktor, Abkühlen des zweiten Reaktorproduktstroms unter seinen Taupunkt und Zuführen des abgekühlten zweiten Reaktorproduktstroms zu einer zweiten Phasentrennvorrichtung,
(h) Auftrennen des abgekühlten zweiten Reaktorproduktstroms in der zweiten Phasentrennvorrichtung in einen zweiten Flüssigproduktstrom und einen zweiten Restgasstrom, der nicht umgesetzte Synthesegasbestandteile enthält,
(i) Auftrennen des zweiten Restgasstroms in einen Spülstrom, der aus dem Verfahren ausgeleitet wird, und in den Recyclestrom, der zu Schritt (b) zurückgeführt wird,
(j) Ausleiten des ersten und des zweiten Flüssigproduktstroms aus dem Verfahren als Rohmethanolproduktstrom, **dadurch gekennzeichnet, dass**
(k) der Frischgas-Bypassstrom in den zweiten Methanolsynthesereaktor eingeleitet wird.

### Erfindungsgemäße Anlage:

Anlage zum Herstellen von Methanol durch Umsetzen eines Wasserstoff und Kohlenoxide enthaltenden Synthesegaseinsatzstroms, umfassend folgende, miteinander in Fluidverbindung stehende Baugruppen und Bestandteile:
(a) Mittel zum Bereitstellen des Wasserstoff und Kohlenoxide enthaltenden Synthesegaseinsatzstroms, Mittel zum Auftrennen des Synthesegaseinsatzstroms in einen Frischgas-Bypassstrom und in einen Frischgas-Feedstrom,
(b) Mittel zum Zusammenführen und Vermischen des Frischgas-Feedstroms mit einem Wasserstoff und Kohlenoxide enthaltenden Recyclestrom zu einem ersten Reaktorfeedstrom,
(c) einen ersten Methanolsynthesereaktor, enthaltend mindestens eine Katalysatorzone mit einem für die Methanolsynthese aktiven, festen, körnigen Katalysator, Mittel zum Einleiten des ersten Reaktorfeedstroms in den ersten Methanolsynthesereaktor,
(d) Mittel zum Ausleiten eines Methanol und Wasser enthaltenden ersten Reaktorproduktstroms aus dem ersten Methanolsynthesereaktor, eine erste Phasentrennvorrichtung, Mittel zum Abkühlen des ersten Reaktorproduktstroms unter seinen Taupunkt und Mittel zum Zuführen des abgekühlten ersten Reaktorproduktstroms zu der ersten Phasentrennvorrichtung,
(e) Mittel zum Auftrennen des abgekühlten ersten Reaktorproduktstroms in der ersten Phasentrennvorrichtung in einen ersten Flüssigproduktstrom und einen ersten Restgasstrom, der nicht umgesetzte Synthesegasbestandteile enthält, Mittel zum Ausleiten des ersten Flüssigproduktstroms, Mittel zum Ausleiten des ersten Restgasstroms,
(f) einen zweiten Methanolsynthesereaktor, enthaltend mindestens eine Katalysatorzone mit einem für die Methanolsynthese aktiven, festen, körnigen Katalysator, Mittel zum Einleiten des ersten Restgasstroms in den zweiten Methanolsynthesereaktor,
(g) Mittel zum Ausleiten eines Methanol und Wasser enthaltenden zweiten Reaktorproduktstroms aus dem zweiten Methanolsynthesereaktor, eine zweite Phasentrennvorrichtung, Mittel zum Abkühlen des zweiten Reaktorproduktstroms unter seinen Taupunkt und Mittel zum Zuführen des abgekühlten zweiten Reaktorproduktstroms zu der zweiten Phasentrennvorrichtung,
(h) Mittel zum Auftrennen des abgekühlten zweiten Reaktorproduktstroms in der zweiten Phasentrennvorrichtung in einen zweiten Flüssigproduktstrom und einen zweiten Restgasstrom, der nicht umgesetzte Synthesegasbestandteile enthält, Mittel zum Ausleiten des zweiten Flüssigproduktstroms, Mittel zum Ausleiten des zweiten Restgasstroms,
(i) Mittel zum Auftrennen des zweiten Restgasstroms in einen Spülstrom und einen Recyclestrom, Mittel zum Ausleiten des Spülstroms aus dem Verfahren, Mittel zum Zurückführen des Recyclestroms zu Baugruppe (b),
(j) Mittel zum Ausleiten des ersten und des zweiten Flüssigproduktstroms aus dem Verfahren als Rohmethanolproduktstrom, **dadurch gekennzeichnet, dass**
(k) Mittel umfasst werden, die es ermöglichen, den Frischgas-Bypassstrom in den zweiten Methanolsynthesereaktor einzuleiten.

Unter Fluidverbindung zwischen zwei Bereichen des erfindungsgemäßen Reaktors wird dabei jegliche Art von Verbindung verstanden, die es ermöglicht, dass ein Fluid, beispielsweise der Einsatzgasstrom oder der Synthesegasproduktstrom, von dem einen zu dem anderen der beiden Bereiche strömen kann, unbeachtlich etwaiger zwischengeschalteter Bereiche oder Bauteile.

Unter Methanolsynthesebedingungen sind die dem Fachmann an sich bekannten Verfahrensbedingungen, insbesondere von Temperatur, Druck und Verweilzeit, zu verstehen, wie sie oben beispielhaft genannt und detailliert im einschlägigen Schrifttum erörtert werden und bei denen mindestens ein Teilumsatz, bevorzugt allerdings technisch relevante Umsätze der Edukte CO bzw. CO₂ und Wasserstoff zum Produkt Methanol erfolgt. Entsprechend wird unter einem für die Methanolsynthese aktiven Katalysator ein Katalysator verstanden, der unter Methanolsynthesebedingungen eben solche Umsätze bewirkt.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile sowie die entsprechenden Durchtrittsöffnungen in Behälterwänden in Betracht ziehen, die ihm aufgrund seines Fachwissens zur Durchführung dieses Verfahrensschrittes notwendig oder sinnvoll erscheinen.

Unter der katalytischen Aktivität, insbesondere im Zusammenhang mit einer unterschiedlichen katalytischen Aktivität beim Vergleich zweier unterschiedlicher Katalysatoren, wird der erzielte Umsatzgrad pro Längeneinheit des Katalysatorbetts von Edukten zu Produkten verstanden. Die Aktivität wird beeinflusst von der chemischen Zusammensetzung, Dotierung, Vergiftung, verfügbaren Oberfläche etc. des Katalysatormaterials, aber auch durch die Geometrie der Katalysatorpartikel und texturellen Parametern des Katalysatorbetts, z. B. dessen Porosität oder Packungsdichte. Aufgrund der Exothermie der betrachteten Reaktionen korreliert eine hohe katalytische Aktivität mit einer hohen Wärmefreisetzung pro Längeneinheit des Katalysatorbetts. Ein weiteres Maß für die Katalysatoraktivität unter festgelegten Methanolsynthesebedingungen stellt der nach jeder Katalysatorstufe mittels Kondensation aufgefangene Mengenstrom an Flüssigprodukten dar, da die Reaktionsprodukte der Methanolsynthesereaktion gemäß der Umsatzgleichungen

CO (g) + 2 H₂ (g) = CH₃OH (I)

CO₂ (g) + 3 H₂ (g) = CH₃OH (I) + H₂O (I)

unter Umgebungsbedingungen flüssig sind. Demzufolge bezeichnet die Größe "Aktivitätsverlust" die zeitliche Abnahme der Katalysatoraktivität und des Edukt-Umsatzgrades bzw. der Methanol-Ausbeute.

Unter einem ersten bzw. zweiten Methanolsynthesereaktor werden nicht notwendigerweise Einzelreaktoren verstanden, sondern es können auch jeweils Gruppen von Einzelreaktoren von diesem Begriff umfasst werden, die ihrerseits wiederum ein oder mehrere Katalysatorzonen, also mit festem, körnigem Methanolsynthesekatalysator gefüllte Bereiche, beinhalten können. "Erster" bzw. "zweiter" Methanolsynthesereaktor soll dabei lediglich die Durchströmungsreihenfolge der betrachteten Reaktoren andeuten. Der erste bzw. zweite Methanolsynthesereaktor müssen nicht zwangsweise direkt aufeinander folgen, sondern es können weitere, nicht näher betrachtete Methanolsynthesereaktoren zwischengeschaltet sein. Kennzeichnend für den zweiten Methanolsynthesereaktor ist es, dass in diesen erfindungsgemäß der Frischgas-Bypassstrom eingeleitet wird.

Unter einem Katalysatorzyklus wird die Betriebsdauer einer Charge eines Methanolsynthesekatalysators verstanden, beginnend mit der Inbetriebnahme des Methanolsynthesebetriebs des mit der Charge frischen oder regenerierten Methanolsynthesekatalysators befüllten Methanolsynthesereaktors und endend mit der Außerbetriebnahme des Methanolsynthesebetriebs desselben Methanolsynthesereaktors zum Zwecke des Katalysatoraustauschs oder der Durchführung der Katalysatorregenerierung.

Der Erfindung liegt die Erkenntnis zugrunde, dass der Aktivitätsverlust eines Methanolsynthesekatalysators infolge Katalysatordesaktivierung durch verschiedene Hauptkriterien verursacht wird, zu denen insbesondere hohe Temperaturen, die zeitliche Produktionsmenge und die Anlagerung von Katalysatorgiften und der Verlust der aktiven Zentren zählen. In mehrstufigen Verfahren bzw. Anlagen mit hintereinandergeschalteten Reaktorsystemen desaktivieren die Katalysatoren in den einzelnen Synthesereaktoren unterschiedlich schnell. So wird beispielsweise der erste Reaktor in Strömungsrichtung stärker belastet, da er mit reaktiverem Synthesegas beaufschlagt wird, das eine höhere Konzentration der Eduktkomponenten enthält. Hieraus resultieren höhere Maximaltemperaturen im Katalysatorbett und eine anfänglich höhere Produktionsrate, die danach jedoch im Vergleich mit nachgeschalteten Reaktoren rascher absinkt. Wenn trotz Ausnutzung möglicher Betriebsparameteranpassungen die vorgeschriebene Gesamtproduktionskapazität der Anlage nicht mehr erreicht wird, werden die Katalysatoren in den Synthesereaktoren ausgetauscht. Der Katalysator im strömungsmäßig ersten Reaktor zeigt dabei zumeist eine stärkere Desaktivierung als der Katalysator in dem oder den nachgeschalteten Reaktoren. Ein separater Austausch nur des Katalysators im ersten Reaktor ist theoretisch möglich, aber logistisch schwierig, und wird daher vermieden. Auch für den Austausch des Katalysators in nur einem Synthesereaktor muss trotzdem die gesamte Syntheseanlage stillgesetzt werden. Es ist daher wenig attraktiv, nur den Katalysator in einem Synthesereaktor auszutauschen, da ansonsten mit weiteren Stillständen für den Austausch der zunächst weniger desaktivierten Katalysatoren in den nachgeschalteten Reaktoren zu rechnen ist.

Aufgrund der erfindungsgemäßen Synthesegaseinspeisung vor einen nachgeschalteten, beispielsweise den zweiten Synthesereaktor werden alle Reaktionszonen gleichmäßiger belastet, woraus eine gleichmäßigere und damit effizientere Ausnutzung des Katalysators resultiert. Dies führt zu einer möglichen Reduzierung des Rückführverhältnis RR sowie des Reaktorvolumens und ferner zu einer verlängerten Katalysatorlebensdauer, höheren Produktionskapazitäten und damit verbunden zu Kostenreduzierungen für den gesamten Prozess. Erreicht wird dies durch die Einspeisung des reaktiven Synthesegases vor einen nachgeschalteten, beispielsweise den zweiten Methanolsynthesereaktor, der somit von Beginn an mit Synthesegas belastet wird, das eine höhere Konzentration an Eduktkomponenten enthält. Der erste Methanolsynthesereaktor wird aufgrund der Verdünnung des Synthesegases mit Recyclegas weniger belastet. Somit kann der Katalysator in dem nachgeschalteten, beispielsweise im zweiten Methanolsynthesereaktor besser ausgenutzt werden und die Synthese effizienter durchgeführt werden. Dies wirkt vorteilhaft mit der Zwischenkondensation der gebildeten Reaktionsprodukte Methanol und Wasser jeweils zwischen zwei Synthesereaktoren zusammen, da die Gasbelastung des oder der nachgeschalteten Synthesereaktoren, die durch die Einspeisung des reaktiven Synthesegases vor eine nachgeschaltete, beispielsweise die zweite Methanolsynthesereaktorstufe ansteigt, durch die Teilkondensation von Produktkomponenten wieder reduziert wird.

Mit zunehmendem Aktivitätsverlust des Katalysators in den Synthesereaktoren kann die Menge an frischem Synthesegas zu einem nachgeschalteten, z. B. zum zweiten Reaktor reduziert werden, um die Verweilzeit des Synthesegases in diesem Synthesereaktor zu erhöhen und somit den zeitlich steigenden Methanol-Ausbeuteverlust zu kompensieren. Ferner kann überraschend mit der erfindungsgemäßen Verschaltung das Rückführverhältnis RR deutlich reduziert werden. Je nach vorgesehener Produktionskapazität und Gaszusammensetzung kann bei Erhalt der Produktionskapazität eine Reduzierung des Rückführverhältnis RR von laut Stand der Technik üblichen Werten zwischen 1,6 und 2,5 auf erfindungsgemäße Werte zwischen 1,0 und 2,0 erfolgen.

Ein wichtiges Kriterium für die Anwendung der erfindungsgemäßen Einspeisung von Frischgas in einen nachgeschalteten Synthesereaktor ist dabei die Temperatursteuerung der Reaktoren und die Methanolproduktion pro Reaktor. Aus diesem Grund bietet die Zwischenkondensation nach jedem Synthesereaktor besondere Vorteile, da der zeitliche Verlauf der Produktmenge pro Reaktor direkt über den Mengenstrom der aufgefangenen, kondensierten Produkte gemessen werden kann, so dass aufwändige Gasanalysen und Durchflussmessungen entfallen.

### Besondere Ausgestaltungen der Erfindung

Eine besondere Ausgestaltung des erfindungsgemäßen Verfahrens ist dadurch gekennzeichnet, dass vor dem Einleiten in den zweiten Methanolsynthesereaktor der Frischgas-Bypassstrom mit dem ersten Restgasstrom zusammengeführt und vermischt wird, wobei ein zweiter Reaktorfeedstrom erhalten wird. Auf diese Weise wird eine gleichmäßige Reaktantenkonzentration am Eingang des zweiten Methanolsynthesereaktor sichergestellt und die Bildung von Konzentrationssträhnen, die zu einem ungleichmäßigen Reaktorbetrieb führen können, vermieden.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus kontinuierlich oder schrittweise verändert wird. Hierdurch wird ein weiterer Freiheitsgrad erhalten, um einen gleichmäßigen Betrieb der Produktionsanlage sicherzustellen und unerwünschten Exkursionen von den Soll-Betriebsparametern, beispielsweise von Temperaturspitzen in den Katalysatorbetten, entgegenzuwirken.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus kontinuierlich oder schrittweise verringert wird. Nach dieser Ausgestaltung wird beispielsweise mit zunehmendem Aktivitätsverlust des Katalysators in den Synthesereaktoren die Menge an frischem Synthesegas zum zweiten Synthesereaktor reduziert, um die Verweilzeit des Synthesegases in diesem Synthesereaktor zu erhöhen und somit den zeitlich steigenden Methanol-Ausbeuteverlust zu kompensieren. Der verbliebene, nicht zum zweiten Synthesereaktor geführte Frischgas-Anteil wird zusätzlich dem ersten Synthesereaktor zugeführt, so dass die Verweilzeit des Synthesegases im ersten Synthesereaktor sinkt, aber über die Summe beider Reaktoren konstant bleibt.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus in Abhängigkeit von dem Aktivitätsverlust des Katalysators im ersten und/oder zweiten Methanolsynthesereaktor kontinuierlich oder schrittweise verringert wird. Nach dieser Ausgestaltung wird demnach mit zunehmendem Aktivitätsverlust des Katalysators in den Synthesereaktoren die Menge an frischem Synthesegas zum zweiten Synthesereaktor reduziert, um die Verweilzeit des Synthesegases in diesem Synthesereaktor zu erhöhen und somit den zeitlich steigenden Methanol-Ausbeuteverlust zu kompensieren. Der verbliebene, nicht zum zweiten Synthesereaktor geführte Frischgas-Anteil wird zusätzlich dem ersten Synthesereaktor zugeführt, so dass die Verweilzeit des Synthesegases im ersten Synthesereaktor sinkt, aber über die Summe beider Reaktoren konstant bleibt. In dieser Ausgestaltung durchläuft ein größerer Anteil des Frischgases beide Reaktoren, so dass trotz des Aktivitätsverlust des Katalysators im ersten und/oder zweiten Methanolsynthesereaktor die Methanolausbeute wieder ansteigt oder aufrecht erhalten wird.

Eine weitere Ausgestaltung des erfindungsgemäßen Verfahrens ist dadurch gekennzeichnet, dass die zeitliche Abnahme des Mengenstroms des ersten und/oder des zweiten Flüssigproduktstroms während eines Katalysatorzyklus als Maß für den Aktivitätsverlust des Katalysators im ersten und/oder zweiten Methanolsynthesereaktor dient. Auf diese Weise kann die Zwischenkondensation nach jedem Synthesereaktor vorteilhaft zu Aktivitätsbestimmung des Katalysators in diesem Reaktor genutzt werden, da der zeitliche Verlauf der Produktmenge pro Reaktor direkt über den Mengenstrom der aufgefangenen, kondensierten Produkte gemessen werden kann, so dass aufwändige Gasanalysen und Durchflussmessungen entfallen.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus kontinuierlich oder schrittweise von einem Anfangswert auf einen Endwert verringert wird, wobei der Anfangswert höchstens 40 Vol.-% des Synthesegaseinsatzstroms entspricht. Untersuchungen zeigen, dass mit diesem Anfangswert eine besonders gute Temperaturkontrolle in beiden Reaktoren und eine besonders günstige Methanolausbeute erhalten werden.

Eine weitere Ausgestaltung des erfindungsgemäßen Verfahrens ist dadurch gekennzeichnet, dass der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird. Der gasgekühlte Reaktor erfüllt somit zwei Funktionen, nämlich als Synthesereaktor und als Wärmetauscher bzw. Feedvorwärmer für den WCR. Eine gute Temperaturkontrolle des zweiten Reaktors, wie sie durch die erfindungsgemäße Frischfeedeinspeisung ermöglicht wird, ist daher bei der Ausgestaltung gemäß WCR-GCR-Konzept besonders wichtig.

Eine weitere Ausgestaltung des erfindungsgemäßen Verfahrens ist dadurch gekennzeichnet, dass der zweite Methanolsynthesereaktor als gasgekühlter Reaktor (GCR) ausgestaltet ist, wobei der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird. Diese Ausgestaltung bietet wegen der verbesserten Kühlleistung am Reaktionsgaseintritt Vorteile. Bei der Gleichstromführung vergrößert sich ferner nahezu der Abstand der Temperatur der Kühlrohrwand von dem Taupunkt des Produktgemischs gegenüber der Gegenstromführung. Damit ist auch beim Betrieb des wassergekühlten ersten Reaktors bei tiefen Temperaturen das Risiko einer Kondensation erheblich geringer. Außerdem ergibt sich eine flachere Temperaturkurve über der Reaktorlänge mit geringeren Spitzentemperaturen. Dies ist vorteilhaft für die Laufzeitstabilität des Katalysators.

Eine besondere Ausgestaltung der erfindungsgemäßen Anlage ist dadurch gekennzeichnet, dass sie ferner Mittel zum Zusammenführen und Vermischen des Frischgas-Bypassstroms mit dem ersten Restgasstrom umfasst. Auf diese Weise wird eine gleichmäßige Reaktantenkonzentration am Eingang des ersten Methanolsynthesereaktor sichergestellt und die Bildung von Konzentrationssträhnen, die zu einem ungleichmäßigen Reaktorbetrieb führen können, vermieden.

Eine weitere vorteilhafte Ausgestaltung der erfindungsgemäßen Anlage ist dadurch gekennzeichnet, dass
- die Mittel zum Ausleiten des ersten und/oder zweiten Flüssigproduktstroms Messvorrichtungen umfassen, die es gestatten, die zeitliche Abnahme des Mengenstroms des ersten und/oder des zweiten Flüssigproduktstroms zu messen,
- die Mittel, die es ermöglichen, den Frischgas-Bypassstrom in den zweiten Methanolsynthesereaktor einzuleiten, ferner mindestens eine Dosiervorrichtung umfassen, die es gestattet, den Mengenstrom des in den zweiten Methanolsynthesereaktor eingeleiteten Frischgas-Bypassstroms kontinuierlich oder schrittweise zu verringern.

Auf diese Weise kann die Zwischenkondensation nach jedem Synthesereaktor vorteilhaft zu Aktivitätsbestimmung des Katalysators in diesem Reaktor genutzt werden, da der zeitliche Verlauf der Produktmenge pro Reaktor direkt über den Mengenstrom der aufgefangenen, kondensierten Produkte gemessen werden kann, so dass aufwändige Gasanalysen und Durchflussmessungen entfallen. Aus der geänderten Katalysatoraktivität kann dann entweder manuell oder bevorzugt mittels eines automatischen Regelsystems der Mengenstrom des in den zweiten Methanolsynthesereaktor eingeleiteten Frischgas-Bypassstroms mit der Dosiervorrichtung kontinuierlich oder schrittweise verringert werden.

Eine weitere vorteilhafte Ausgestaltung der erfindungsgemäßen Anlage ist dadurch gekennzeichnet, dass der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei ferner Mittel umfasst werden, die es gestatten, dass der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird. Der gasgekühlte Reaktor erfüllt somit zwei Funktionen, nämlich als Synthesereaktor und als Wärmetauscher bzw. Feedvorwärmer für den WCR. Eine gute Temperaturkontrolle des zweiten Reaktors, wie sie durch die erfindungsgemäße Frischfeedeinspeisung ermöglicht wird, ist daher bei der Ausgestaltung gemäß WCR-GCR-Konzept besonders wichtig.

Ein weiterer Aspekt der erfindungsgemäßen Anlage ist dadurch gekennzeichnet, dass der zweite Methanolsynthesereaktor als gasgekühlter Reaktor (GCR) ausgestaltet ist und ferner so ausgestattet ist, dass es ermöglicht wird, dass der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird. Diese Ausgestaltung bietet wegen der verbesserten Kühlleistung am Reaktionsgaseintritt Vorteile. Bei der Gleichstromführung vergrößert sich ferner nahezu der Abstand der Temperatur der Kühlrohrwand von dem Taupunkt des Produktgemischs gegenüber der Gegenstromführung. Damit ist auch beim Betrieb des wassergekühlten ersten Reaktors bei tiefen Temperaturen das Risiko einer Kondensation erheblich geringer. Außerdem ergibt sich eine flachere Temperaturkurve über der Reaktorlänge mit geringeren Spitzen-temperaturen. Dies ist vorteilhaft für die Laufzeitstabilität des Katalysators.

Ein weiterer Aspekt der erfindungsgemäßen Anlage ist dadurch gekennzeichnet, dass der zweite Methanolsynthesereaktor als gasgekühlter Reaktor (GCR) ausgestaltet ist und einen Reaktormantel und in dessen Inneren eine Vielzahl von Rohren umfasst, wobei der für die Methanolsynthese aktive, feste, körnige Katalysator entweder in den Rohren oder in dem Zwischenraum zwischen der Innenseite des Reaktormantels und der Außenseite der Rohre angeordnet ist und wobei der erste Reaktorfeedstrom als Kühlgas durch den jeweils anderen, nicht Katalysator enthaltenden Bereich geleitet wird. Besonders bevorzugt wird dabei der Katalysator in dem Zwischenraum zwischen der Innenseite des Reaktormantels und der Außenseite der Rohre angeordnet und die Rohre von dem ersten Reaktorfeedstrom als Kühlgas durchströmt.

### Ausführungs- und Zahlenbeispiele

Weiterbildungen, Vorteile und Anwendungsmöglichkeiten der Erfindung ergeben sich auch aus der nachfolgenden Beschreibung von Ausführungs- und Zahlenbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigen:
- Fig. 1: eine schematische Darstellung des Verfahrens bzw. der Anlage gemäß einer ersten Ausführungsform der Erfindung mit zwei wassergekühlten Reaktoren,
- Fig. 2: eine schematische Darstellung des Verfahrens bzw. der Anlage gemäß einer zweiten Ausführungsform der Erfindung mit einem wassergekühlte Reaktor als erster Reaktor und einem gasgekühlten Reaktor als zweiter Reaktor.

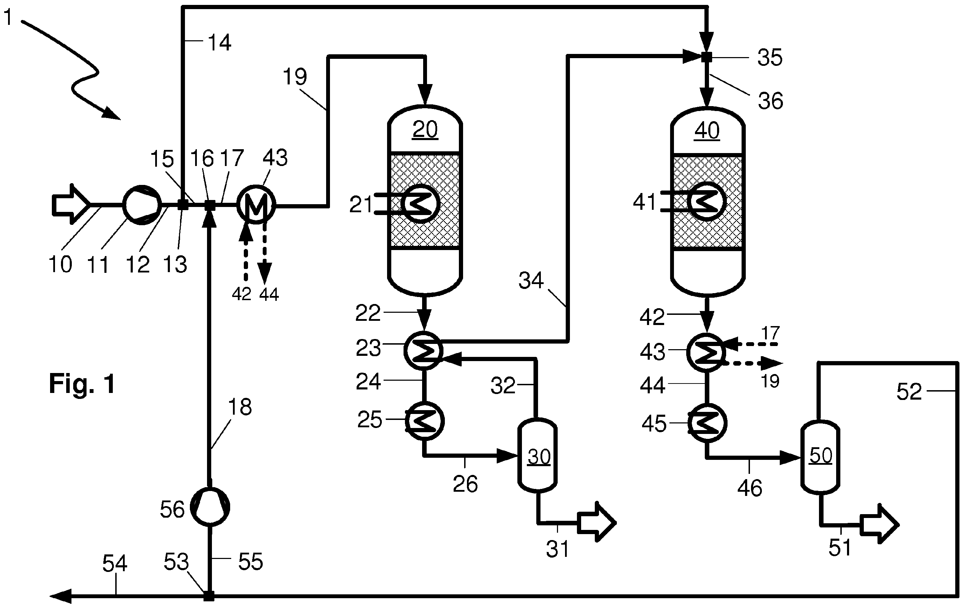

In der in Fig. 1 dargestellten, ersten Ausgestaltung eines Verfahrens 1 bzw. einer Anlage 1 gemäß der Erfindung, die zwei hintereinander geschaltete, wassergekühlte Synthesereaktoren 20, 40 umfasst, wird über Leitung 10 frisches Synthesegas (Frischgas, Make-up-Gas), enthaltend Wasserstoff, Kohlenmonoxid und Kohlendioxid, aus einer nicht dargestellten Synthesegaserzeugungsanlage über Leitung 10 eingeleitet, mittels Verdichter 11 auf Synthesedruck verdichtet und über Leitung 12 zu einer Auftrennvorrichtung 13 geführt, die beispielsweise als T-Rohrstück ausgestaltet sein kann. Aus der Auftrennvorrichtung wird über Leitung 14 ein Teilstrom des Frischgases ausgeleitet und als Frischgas-Bypassstrom zum zweiten Synthesereaktor 40 geführt. Im Verlauf der Leitung 14 kann eine bildlich nicht dargestellte Dosiervorrichtung angeordnet sein, mit deren Hilfe der Mengenstrom des Frischgas-Bypassstroms eingestellt werden kann.

Der verbleibende Anteil des Frischgases wird über Leitung 15 als Frischgas-Feedstrom zu Mischvorrichtung 16 geleitet und in dieser mit einem Recyclestrom zusammengeführt, der über Leitung 18 herangeführt und ebenfalls in die Mischvorrichtung 16 eingeleitet wird. Die Mischvorrichtung 16 kann, wie auch die nachfolgend genannten Mischvorrichtungen, zum Beispiel als T-Rohrstück oder als statischer Mischer ausgestaltet sein. Das Verhältnis der Mengenströme, die mittels der Leitungen 18 (Recyclestrom) und 15 (Frischgas) zu der Mischvorrichtung 16 geführt werden, entspricht dem Rückführverhältnis RR.

Durch das Zusammenführen und Vermischen des Frischgas-Feedstroms mit dem Recyclestrom wird ein erster Reaktorfeedstrom erhalten, der über Leitung 17 zu Wärmetauscher 43 geführt wird und in diesem im indirekten Wärmetausch gegen den heißen Reaktorproduktstrom aus dem zweiten Synthesereaktor 40 auf die Reaktoreintrittstemperatur aufgeheizt wird. Er wird sodann über Leitung 19 in den ersten Methanolsynthesereaktor 20 eingeleitet.

Im ersten Methanolsynthesereaktor 20, der mindestens eine Katalysatorzone mit einem einen für die Methanolsynthese aktiven, festen, körnigen Katalysator enthält, erfolgt die Teilumsetzung des ersten Reaktorfeedstroms unter Methanolsynthesebedingungen. Im Ausführungsbeispiel der Fig. 1 sind beide Synthesereaktoren 20, 40 wassergekühlt; die jeweiligen in die Reaktoren integrierten Kühlvorrichtungen sind mittels Bezugszeichen 21, 41 angedeutet.

Aus dem ersten Synthesereaktor 20 wird über Leitung 22 ein heißer erster Reaktorproduktstrom ausgeleitet und zu Wärmetauscher 23 und sodann über Leitung 24 zu Kühler 25 geführt. In Wärmetauscher 23 erfolgt eine erste Abkühlung des heißen ersten Reaktorproduktstroms durch indirekten Wärmetausch gegen den aus der ersten Phasentrennvorrichtung 30 ausgeleiteten, abgekühlten Restgasstrom. Kühler 25 kann beispielsweise als Luftkühler oder als mit Kühlwasser betriebener Kühler ausgestaltet werden. Der unter seinen Taupunkt abgekühlte, erste Reaktorproduktstrom wird über Leitung 26 in eine erste Phasentrennvorrichtung 30 eingeleitet und in dieser in einen ersten Flüssigproduktstrom und in einen ersten Restgasstrom aufgetrennt. Der erste Flüssigproduktstrom, der im Wesentlichen Methanol und Wasser enthält, wird über Leitung 31 aus dem Verfahren bzw. aus der Anlage ausleitet und einer bildlich nicht dargestellten Rohmethanolaufarbeitung zugeführt. Der erste Restgasstrom, der noch nicht umgesetzte Synthesegasbestandteile enthält, wird über Leitung 32 zu Wärmetauscher 23 geführt und in diesem im indirekten Wärmetausch gegen den heißen ersten Reaktorproduktstrom aus dem Methanolsynthesereaktor 20 aufgeheizt. Anschließend wird der nunmehr aufgeheizte erste Restgasstrom über Leitung 34 zu Mischvorrichtung 35 geführt.

In Mischvorrichtung 35 wird der aufgeheizte erste Restgasstrom mit dem über Leitung 14 herangeführten Frischgas-Bypassstrom zusammengeführt und vermischt. Auch die Mischvorrichtung 35 kann beispielsweise als T-Rohrstück oder als statischer Mischer ausgestaltet sein. Der dabei erhaltene zweite Reaktorfeedstrom wird sodann mittels Leitung 36 in den zweiten Methanolsynthesereaktor 40 eingeleitet, der ebenfalls mindestens eine Katalysatorzone mit einem einen für die Methanolsynthese aktiven, festen, körnigen Katalysator enthält. Im zweiten Methanolsynthesereaktor erfolgt die Teilumsetzung des zweiten Reaktorfeedstroms unter Methanolsynthesebedingungen.

Aus dem zweiten Methanolsynthesereaktor 40 wird über Leitung 42 ein heißer zweiter Reaktorproduktstrom ausgeleitet und zu Wärmetauscher 43 und sodann über Leitung 44 zu Kühler 45 geführt. Im Wärmetauscher 43 erfolgt eine erste Abkühlung des heißen zweiten Reaktorproduktstroms durch indirekten Wärmetausch gegen den über Leitung 17 herangeführten erster Reaktorfeedstrom. Kühler 45 kann ebenfalls beispielsweise als Luftkühler oder als mit Kühlwasser betriebener Kühler ausgestaltet werden. Der unter seinen Taupunkt abgekühlte, zweite Reaktorproduktstrom wird über Leitung 46 in eine zweite Phasentrennvorrichtung 50 eingeleitet und in dieser in einen zweiten Flüssigproduktstrom und in einen zweiten Restgasstrom aufgetrennt. Der zweite Flüssigproduktstrom, der wiederum im Wesentlichen Methanol und Wasser enthält, wird über Leitung 51 aus dem Verfahren bzw. aus der Anlage ausleitet und der bildlich nicht dargestellten Rohmethanolaufarbeitung zugeführt. Der zweite Restgasstrom, der noch nicht umgesetzte Synthesegasbestandteile enthält, wird über Leitung 52 zu Auftrennvorrichtung 53 geführt.

In Auftrennvorrichtung 53, die die beispielsweise als T-Rohrstück ausgestaltet sein kann, erfolgt die Auftrennung des zweiten Restgasstroms in einen Spülstrom, der aus dem Verfahren bzw. der Anlage mittels Leitung 54 ausgeleitet wird, und in einen Recyclestrom, der mittels Leitung 55 zu Verdichter 56 geführt wird. Das Ausleiten des Spülstroms über Leitung 54 dient dazu, die Akkumulation von Inertkomponenten wie z. B. Argon oder Methan innerhalb des Synthesekreislaufs zu verhindern. Zum Einstellen des Spülstrom-Mengenstroms kann im Leitungsweg der Leitung 54 ein nicht dargestelltes Dosierventil vorgesehen werden.

Der verdichtete Recyclestrom wird über Leitung 18 aus Verdichter 56 ausgeleitet und in Mischvorrichtung 16 eingeleitet.

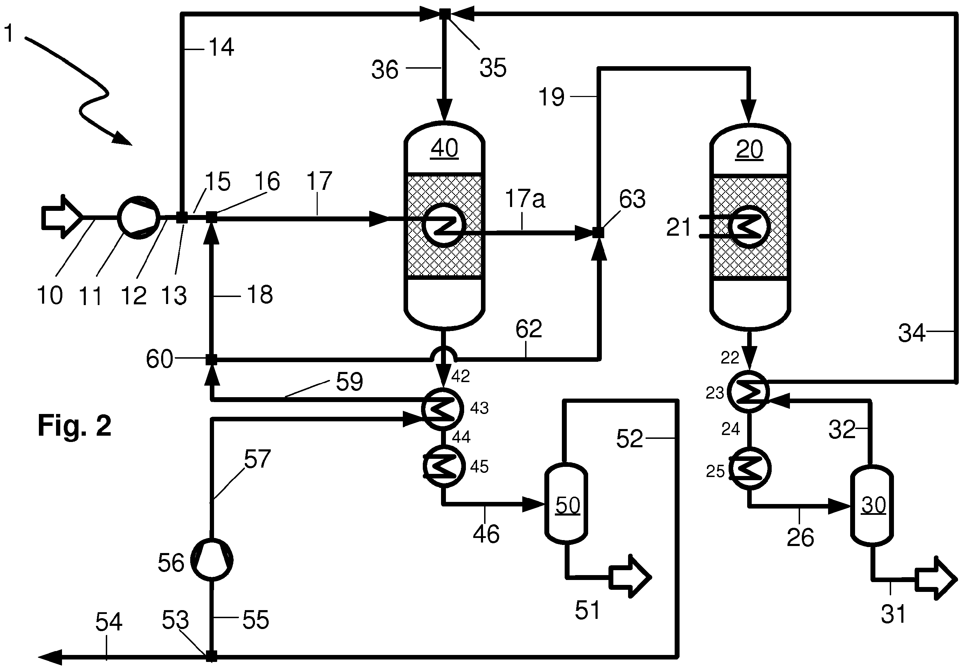

In der in Fig. 2 dargestellten, zweiten Ausgestaltung der Erfindung umfasst das Verfahren 1 bzw. die Anlage 1 einen wassergekühlten Synthesereaktor 20 (WCR) und einen gasgekühlten Synthesereaktor 40 (GCR). Durch gleiche Bezugszeichen gekennzeichnete Zeichnungselemente entsprechen in ihrer Funktion und Beschaffenheit denen, die im Zusammenhang mit Fig. 1 erläutert wurden, sofern im Einzelfall nichts anderes angegeben ist.

Im Vergleich zu der ersten Ausgestaltung ergeben sich für die in Fig. 2 gezeigte Ausgestaltung der Erfindung folgende Unterschiede:
Der erste Reaktorfeedstrom wird über Leitung 17 zunächst zum gasgekühlten Reaktor 40 (GCR), dem zweiten Methanolsynthesereaktor geführt, durchläuft dort die in ihn integrierte Wärmeaustauschvorrichtung (bildlich angedeutet durch den in den Reaktor eingezeichneten Wärmetauscher) und wird dort im indirekten Wärmetausch gegen die heißen Reaktorproduktgase aufgeheizt. Gleichzeitig dient der erste Reaktorfeedstrom somit als Kühlgasstrom im Reaktor 40. Der in Fig. 1 eingezeichnete Wärmetauscher 43 kann im Normalfall entfallen und ist deshalb bildlich in Fig. 2 nicht dargestellt, optional kann er jedoch als Reserve oder zur Realisierung besonderer Betriebszustände, beispielsweise zur Inbetriebnahme der Anlage, vorgesehen werden. Der aufheizte erste Reaktorfeedstrom wird dann über Leitung 17a, Mischvorrichtung 63 und Leitung 19 zum wassergekühlten Reaktor 20 (WCR) als erstem Methanolsynthesereaktor geführt und diesem aufgegeben. Über Leitung 62 kann noch ein Teilstrom des Recyclestroms, das mittels Auftrennvorrichtung 60 abgezweigt wurde, dem ersten Reaktorfeedstrom zur Temperatureinstellung mit Hilfe von Mischvorrichtung 63 zugemischt werden. Die Einstellung des Teilstrom-Mengenstroms erfolgt beispielsweise mit einer nicht gezeigten, in die Leitung 62 integrierten Dosiervorrichtung.

Die Kühlung des aus dem zweiten Methanolsynthesereaktor 40 über Leitung 42 abgeführten, heißen zweiten Reaktorproduktstroms im Wärmetauscher 43 erfolgt im Unterschied zu Fig. 1 mittels des in Leitung 57 herangeführten Recyclestroms als Kühlgas. Über Leitung 59 wird der aufgeheizte Recyclestrom aus dem Wärmetauscher 43 abgeführt und über Auftrennvorrichtung 60, Leitung 18, Mischvorrichtung 16, Leitung 17, 17a, Mischvorrichtung 63 und Leitung 19 zum ersten Methanolsynthesereaktor 20 zurückgeführt.

### Zahlenbeispiel 1

In einer zweistufigen Methanolsyntheseanlage, ausgestattet mit einem wassergekühlten Reaktor (WCR) als erstem Methanolsynthesereaktor und einem gasgekühlten Reaktor (GCR) als zweitem Methanolsynthesereaktor wurde Synthesegas, erhalten aus Erdgas mittels mehrstufiger Reformierung durch Steamreforming und Autothermreforming (sog. Combined Reforming) wurden Betriebsversuche zur Methanolsynthese durchgeführt. Beide Reaktoren wiesen dieselbe Reaktorgeometrie auf und enthielten jeweils 80 m³ eines vom Handel bezogenen, festen, körnigen Katalysators für die Methanolsynthese auf Kupferbasis als Festbettschüttung. Nach jeder der beiden Reaktorstufen erfolgte eine Kondensation der Reaktionsprodukte. Die Betriebsversuche gemäß der Erfindung wurden mit einem konstanten Bypassstrom zum zweiten Synthesereaktor durchgeführt, der 20 % des Frischgasstroms entsprach. Bei den Vergleichsversuchen war die Bypassleitung zum zweiten Synthesereaktor geschlossen. Die Zusammensetzung und der Mengenstrom des Frischgases, der Mengenstrom des Recyclestroms und der Reaktordruck waren bei den erfindungsgemäßen Betriebsversuchen und den Vergleichsversuchen gleich.

In Tabelle 1 sind Ergebnisse dieser Betriebsversuche zusammengestellt, unterschieden nach Beginn (start of run, SOR) und Ende (end of run, EOR) eines Katalysatorzyklus bestimmter Dauer. Anhand der wiedergegebenen Raum-Zeit-Ausbeuten (STY, kg/h Methanolprodukt pro Liter Katalysator) ist erkennbar, dass mit der Erfindung die Katalysatorbelastung im ersten Synthesereaktor abgesenkt und zur Kompensation im zweiten Synthesereaktor erhöht werden kann. Dies ermöglich eine gleichmäßigere Katalysatorbelastung und somit eine langsamere Katalysatordesaktivierung bzw. längere Katalysatorzyklen.

| **Tabelle 1** | | Vgl.bsp. | Vgl.bsp. | Erfindung | Erfindung |
|---|---|---|---|---|---|
| Setup | | WCR + GCR | WCR + GCR | WCR + GCR | WCR + GCR |
| Bypass | | NO | NO | YES | YES |
| Fall Katalysatorzyklus | | SOR | EOR | SOR 2 | EOR2 |
| Synthesegas | kmol/h | 10798 | 10798 | 10798 | 10798 |
| Produktion | kmol/h | 2346 | 2331 | 2331 | 2313 |
| Recvcle | kmol/h | 18357 | 18357 | 18357 | 18357 |
| RR | | 1,7 | 1,7 | 1,7 | 1,7 |
| Split Bypass | % | 0 | 0 | 20 | 20 |
| STY 1 | (kg/h) / L | 1,48 | 1,336 | 1,255 | 1,195 |
| STY2 | (kg/h) / L | 0,453 | 0,57 | 0,644 | 0,685 |

### Zahlenbeispiel 2

In einer zweiten Reihe von Betriebsversuchen wurde ein durch Kohlevergasung erhaltenes Synthesegas als Frischgas eingesetzt. Die Katalysatorvolumina betrugen nun 100 m³ im ersten und 76 m³ im zweiten Synthesereaktor. Der Aufbau der Syntheseanlage war ansonsten wie oben beschrieben. Das Rückführverhältnis RR wurde während eines Katalysatorzyklus von 1,5 auf 1,9 erhöht. Weitere Betriebsparameter und Versuchsergebnisse sind in Tabelle 2 zusammengestellt, wiederum unterschieden nach Beginn (start of run, SOR) und Ende (end of run, EOR) eines Katalysatorzyklus bestimmter Dauer. Bei den erfindungsgemäßen Versuchen wurde nun während des Katalysatorzyklus der Mengenstrom des Frischgas-Bypassstroms zum zweiten Synthesereaktor abgesenkt. Trotzdem ergibt sich auch nach dieser Absenkung eine geringere Belastung des ersten und eine höhere Belastung des zweiten Synthesereaktors und somit eine gleichmäßigere Katalysatorbelastung über die Syntheseanlage.

| **Tabelle 2** | - | Vgl.bsp. | Vgl.bsp. | Erfindung | Erfindung |
|---|---|---|---|---|---|
| Setup | | WCR + GCR | WCR + GCR | WCR + GCR | WCR + GCR |
| Bypass | | NO | NO | YES | YES |
| Fall Katalysatorzyklus | | SOR | EOR | SOR | EOR |
| Syntheseqas | kmol/h | 24800 | 24850 | 24800 | 24850 |
| Produktion | t/d | 6080 | 6061 | 6077 | 6069 |
| RR | - | 1,50 | 1,90 | 1,50 | 1,90 |
| Split Bypass | %/100 | 0,00 | 0,00 | 0,15 | 0,05 |
| STY R1 | (kg/h) / L | 1,85 | 1,6 | 1,7 | 1,56 |
| STY R2 | (kg/h) / L | 0,9 | 1,22 | 1,09 | 1,27 |

### Bezugszeichenliste

- [1]: Verfahren, Anlage
- [10]: Leitung
- [11]: Verdichter
- [12]: Leitung
- [13]: Auftrennvorrichtung
- [14]: Leitung
- [15]: Leitung
- [16]: Mischvorrichtung
- [17]: Leitung
- [18]: Leitung
- [19]: Leitung
- [20]: erster Methanolsynthesereaktor
- [21]: Kühlvorrichtung
- [22]: Leitung
- [23]: Wärmetauscher
- [24]: Leitung
- [25]: Kühler
- [26]: Leitung
- [30]: erste Phasentrennvorrichtung
- [31]: Leitung
- [32]: Leitung
- [34]: Leitung
- [35]: Mischvorrichtung
- [36]: Leitung
- [40]: zweiter Methanolsynthesereaktor
- [41]: Kühlvorrichtung
- [42]: Leitung
- [43]: Wärmetauscher
- [44]: Leitung
- [45]: Kühler
- [46]: Leitung
- [50]: zweite Phasentrennvorrichtung
- [51]: Leitung
- [52]: Leitung
- [53]: Auftrennvorrichtung
- [54]: Leitung
- [55]: Leitung
- [56]: Verdichter
- [57]: Leitung
- [59]: Leitung
- [60]: Auftrennvorrichtung
- [62]: Leitung
- [63]: Mischvorrichtung

## Patentansprüche

1. Verfahren zum Herstellen von Methanol durch Umsetzen eines Wasserstoff und Kohlenoxide enthaltenden Synthesegaseinsatzstroms, umfassend folgende Verfahrensschritte:
(a) Bereitstellen des Wasserstoff und Kohlenoxide enthaltenden Synthesegaseinsatzstroms, Auftrennen des Synthesegaseinsatzstroms in einen Frischgas-Bypassstrom und in einen Frischgas-Feedstrom,
(b) Zusammenführen und Vermischen des Frischgas-Feedstroms mit einem Wasserstoff und Kohlenoxide enthaltenden Recyclestrom zu einem ersten Reaktorfeedstrom,
(c) Einleiten des ersten Reaktorfeedstroms in einen ersten Methanolsynthesereaktor, enthaltend mindestens eine Katalysatorzone mit einem einen für die Methanolsynthese aktiven, festen, körnigen Katalysator, mindestens teilweises Umsetzen des ersten Reaktorfeedstroms in dem ersten Methanolsynthesereaktor unter Methanolsynthesebedingungen,
(d) Ausleiten eines Methanol und Wasser enthaltenden ersten Reaktorproduktstroms aus dem ersten Methanolsynthesereaktor, Abkühlen des ersten Reaktorproduktstroms unter seinen Taupunkt und Zuführen des abgekühlten ersten Reaktorproduktstroms zu einer ersten Phasentrennvorrichtung,
(e) Auftrennen des abgekühlten ersten Reaktorproduktstroms in der ersten Phasentrennvorrichtung in einen ersten Flüssigproduktstrom und einen ersten Restgasstrom, der nicht umgesetzte Synthesegasbestandteile enthält,
(f) Einleiten des ersten Restgasstroms in einen zweiten Methanolsynthesereaktor, enthaltend mindestens eine Katalysatorzone mit einem einen für die Methanolsynthese aktiven, festen, körnigen Katalysator, mindestens teilweises Umsetzen des ersten Restgasstroms in dem zweiten Methanolsynthesereaktor unter Methanolsynthesebedingungen,
(g) Ausleiten eines Methanol und Wasser enthaltenden zweiten Reaktorproduktstroms aus dem zweiten Methanolsynthesereaktor, Abkühlen des zweiten Reaktorproduktstroms unter seinen Taupunkt und Zuführen des abgekühlten zweiten Reaktorproduktstroms zu einer zweiten Phasentrennvorrichtung,
(h) Auftrennen des abgekühlten zweiten Reaktorproduktstroms in der zweiten Phasentrennvorrichtung in einen zweiten Flüssigproduktstrom und einen zweiten Restgasstrom, der nicht umgesetzte Synthesegasbestandteile enthält,
(i) Auftrennen des zweiten Restgasstroms in einen Spülstrom, der aus dem Verfahren ausgeleitet wird, und in den Recyclestrom, der zu Schritt (b) zurückgeführt wird,
(j) Ausleiten des ersten und des zweiten Flüssigproduktstroms aus dem Verfahren als Rohmethanolproduktstrom, **dadurch gekennzeichnet, dass**
(k) der Frischgas-Bypassstrom in den zweiten Methanolsynthesereaktor eingeleitet wird.

2. Verfahren nach Anspruch 1, **dadurch gekennzeichnet, dass** vor dem Einleiten in den zweiten Methanolsynthesereaktor der Frischgas-Bypassstrom mit dem ersten Restgasstrom zusammengeführt und vermischt wird, wobei ein zweiter Reaktorfeedstrom erhalten wird.

3. Verfahren nach Anspruch 1 oder 2, **dadurch gekennzeichnet, dass** der Mengenstrom des in den zweiten Methanolsynthesereaktor eingeleiteten Frischgas-Bypassstroms während eines Katalysatorzyklus kontinuierlich oder schrittweise verändert wird.

4. Verfahren nach Anspruch 3, **dadurch gekennzeichnet, dass** der Mengenstrom des in den zweiten Methanolsynthesereaktor eingeleiteten Frischgas-Bypassstroms während eines Katalysatorzyklus kontinuierlich oder schrittweise verringert wird.

5. Verfahren nach Anspruch 4, **dadurch gekennzeichnet, dass** der Mengenstrom des in den zweiten Methanolsynthesereaktor eingeleiteten Frischgas-Bypassstroms während eines Katalysatorzyklus in Abhängigkeit von dem Aktivitätsverlust des Katalysators im ersten und/oder zweiten Methanolsynthesereaktor kontinuierlich oder schrittweise verringert wird.

6. Verfahren nach Anspruch 4, **dadurch gekennzeichnet, dass** die zeitliche Abnahme des Mengenstroms des ersten und/oder des zweiten Flüssigproduktstroms während eines Katalysatorzyklus als Maß für den Aktivitätsverlust des Katalysators im ersten und/oder zweiten Methanolsynthesereaktor dient.

7. Verfahren nach einem der Ansprüche 4 bis 6, **dadurch gekennzeichnet, dass** der Mengenstrom des in den zweiten Methanolsynthesereaktor eingeleiteten Frischgas-Bypassstroms während eines Katalysatorzyklus kontinuierlich oder schrittweise von einem Anfangswert auf einen Endwert verringert wird, wobei der Anfangswert höchstens 40 Vol.-% des Synthesegaseinsatzstroms entspricht.

8. Verfahren nach einem der vorigen Ansprüche, **dadurch gekennzeichnet, dass** der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird.

9. Verfahren nach Anspruch 8, **dadurch gekennzeichnet, dass** der zweite Methanolsynthesereaktor als gasgekühlter Reaktor (GCR) ausgestaltet ist, wobei der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird.

10. Anlage zum Herstellen von Methanol durch Umsetzen eines Wasserstoff und Kohlenoxide enthaltenden Synthesegaseinsatzstroms, umfassend folgende, miteinander in Fluidverbindung stehende Baugruppen und Bestandteile:
(a) Mittel zum Bereitstellen des Wasserstoff und Kohlenoxide enthaltenden Synthesegaseinsatzstroms, Mittel zum Auftrennen des Synthesegaseinsatzstroms in einen Frischgas-Bypassstrom und in einen Frischgas-Feedstrom,
(b) Mittel zum Zusammenführen und Vermischen des Frischgas-Feedstroms mit einem Wasserstoff und Kohlenoxide enthaltenden Recyclestrom zu einem ersten Reaktorfeedstrom,
(c) einen ersten Methanolsynthesereaktor, enthaltend mindestens eine Katalysatorzone mit einem für die Methanolsynthese aktiven, festen, körnigen Katalysator, Mittel zum Einleiten des ersten Reaktorfeedstroms in den ersten Methanolsynthesereaktor,
(d) Mittel zum Ausleiten eines Methanol und Wasser enthaltenden ersten Reaktorproduktstroms aus dem ersten Methanolsynthesereaktor, eine erste Phasentrennvorrichtung, Mittel zum Abkühlen des ersten Reaktorproduktstroms unter seinen Taupunkt und Mittel zum Zuführen des abgekühlten ersten Reaktorproduktstroms zu der ersten Phasentrennvorrichtung,
(e) Mittel zum Auftrennen des abgekühlten ersten Reaktorproduktstroms in der ersten Phasentrennvorrichtung in einen ersten Flüssigproduktstrom und einen ersten Restgasstrom, der nicht umgesetzte Synthesegasbestandteile enthält, Mittel zum Ausleiten des ersten Flüssigproduktstroms, Mittel zum Ausleiten des ersten Restgasstroms,
(f) einen zweiten Methanolsynthesereaktor, enthaltend mindestens eine Katalysatorzone mit einem für die Methanolsynthese aktiven, festen, körnigen Katalysator, Mittel zum Einleiten des ersten Restgasstroms in den zweiten Methanolsynthesereaktor,
(g) Mittel zum Ausleiten eines Methanol und Wasser enthaltenden zweiten Reaktorproduktstroms aus dem zweiten Methanolsynthesereaktor, eine zweite Phasentrennvorrichtung, Mittel zum Abkühlen des zweiten Reaktorproduktstroms unter seinen Taupunkt und Mittel zum Zuführen des abgekühlten zweiten Reaktorproduktstroms zu der zweiten Phasentrennvorrichtung,
(h) Mittel zum Auftrennen des abgekühlten zweiten Reaktorproduktstroms in der zweiten Phasentrennvorrichtung in einen zweiten Flüssigproduktstrom und einen zweiten Restgasstrom, der nicht umgesetzte Synthesegasbestandteile enthält, Mittel zum Ausleiten des zweiten Flüssigproduktstroms, Mittel zum Ausleiten des zweiten Restgasstroms,
(i) Mittel zum Auftrennen des zweiten Restgasstroms in einen Spülstrom und einen Recyclestrom, Mittel zum Ausleiten des Spülstroms aus dem Verfahren, Mittel zum Zurückführen des Recyclestroms zu Baugruppe (b),
(j) Mittel zum Ausleiten des ersten und des zweiten Flüssigproduktstroms aus dem Verfahren als Rohmethanolproduktstrom, **dadurch gekennzeichnet, dass**
(k) Mittel umfasst werden, die es ermöglichen, den Frischgas-Bypassstrom in den zweiten Methanolsynthesereaktor einzuleiten.

11. Anlage nach Anspruch 10, ferner umfassend Mittel zum Zusammenführen und Vermischen des Frischgas-Bypassstroms mit dem ersten Restgasstrom.

12. Anlage nach Anspruch 10 oder 11, **dadurch gekennzeichnet, dass**
- die Mittel zum Ausleiten des ersten und/oder zweiten Flüssigproduktstroms Messvorrichtungen umfassen, die es gestatten, die zeitliche Abnahme des Mengenstroms des ersten und/oder des zweiten Flüssigproduktstroms zu messen,
- die Mittel, die es ermöglichen, den Frischgas-Bypassstrom in den zweiten Methanolsynthesereaktor einzuleiten, ferner mindestens eine Dosiervorrichtung umfassen, die es gestattet, den Mengenstrom des in den zweiten Methanolsynthesereaktor eingeleiteten Frischgas-Bypassstroms kontinuierlich oder schrittweise zu verringern.

13. Anlage nach einem der vorigen Ansprüche, **dadurch gekennzeichnet, dass** der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei ferner Mittel umfasst werden, die es gestatten, dass der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird.

14. Anlage nach Anspruch 13, **dadurch gekennzeichnet, dass** der zweite Methanolsynthesereaktor als gasgekühlter Reaktor (GCR) ausgestaltet ist und ferner so ausgestattet ist, dass es ermöglicht wird, dass der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird.

15. Anlage nach Anspruch 13 oder 14, **dadurch gekennzeichnet, dass** der zweite Methanolsynthesereaktor als gasgekühlter Reaktor (GCR) ausgestaltet ist und einen Reaktormantel und in dessen Inneren eine Vielzahl von Rohren umfasst, wobei der für die Methanolsynthese aktive, feste, körnige Katalysator entweder in den Rohren oder in dem Zwischenraum zwischen der Innenseite des Reaktormantels und der Außenseite der Rohre angeordnet ist und wobei der erste Reaktorfeedstrom als Kühlgas durch den jeweils anderen, nicht Katalysator enthaltenden Bereich geleitet wird.
